# EUROPEAN PATENT APPLICATION

(11) **EP 1 374 888 A1**
(43) Date of publication of application: **02.01.2004**
(21) Application number: 02701642.7
(22) Date of filing: 28.02.2002
(51) Int. Cl.: A61K 38/17, A61K 31/711, A61K 48/00, A61K 38/57, A61P 19/02, A61P 29/00, A61P 43/00, C07K 14/47, C12N 15/09

(54) **REMEDIES FOR ARTHRITIS DEFORMANS AND REMEDIES FOR RHEUMATOID ARTHRITIS**

(30) Priority: 28.02.2001 JP 2001055305
(71) Applicant: Mitsubishi Pharma Corporation, Osaka-shi, Osaka 541-0046 (JP)
(72) Inventor: IMAIZUMI, Rei, MITSUBISHI PHARMA CORPORATION,, Chuo-ku, Tokyo 103-8405 (JP)
(74) Representative: von Kreisler, Alek, Dipl.-Chem.
(86) International application number: PCT/JP2002/001865
(87) International publication number: WO 2002/072134

(57) **Abstract**

The present invention aims at providing a therapeutic agent effective for diseases of osteoarthritis and chronic rheumatoid arthritis, and provides a therapeutic agent for osteoarthritis or therapeutic agent for chronic rheumatoid arthritis which contains, as an active ingredient, a chondromodulin-I protein having an activity of growing articular chondrocytes by itself or under coexistence of a basic fibroblast growth factor and an activity of suppressing hyper degradation of cartilage matrix.

SEQUENCE LISTING

<110> Mitsubishi Pharma Corporation

<120> Therapeutic agent for osteoarthritis deformans and
therapeutic agent for chronic rheumatoid arthritis

<130> 02008WO-1326

<150> JP 2001-055305
<151> 2001-02-28

<160> 6

<210> 1
<211> 1006
<212> DNA
<213> Homo sapiens

<220>
<221> CDS
<222> (2)..(1003)

<400> 1
c atg aca gag aac tcc gac aaa gtt ccc att gcc ctg gtg gga cct gat 49
  Met Thr Glu Asn Ser Asp Lys Val Pro Ile Ala Leu Val Gly Pro Asp
  1               5                   10                  15
gac gtg gaa ttc tgc agc ccc ccg gcg tac gct acg ctg acg gtg aag   97
Asp Val Glu Phe Cys Ser Pro Pro Ala Tyr Ala Thr Leu Thr Val Lys
            20                  25                  30
ccc tcc agc ccc gcg cgg ctg ctc aag gtg gga gcc gtg gtc ctc att   145
Pro Ser Ser Pro Ala Arg Leu Leu Lys Val Gly Ala Val Val Leu Ile
        35                  40                  45
tcg gga gct gtg ctg ctg ctc ttt ggg gcc atc ggg gcc ttc tac ttc   193
Ser Gly Ala Val Leu Leu Leu Phe Gly Ala Ile Gly Ala Phe Tyr Phe
    50                  55                  60
tgg aag ggg agc gac agt cac att tac aat gtc cat tac acc atg agt   241
Trp Lys Gly Ser Asp Ser His Ile Tyr Asn Val His Tyr Thr Met Ser
65                  70                  75                  80
atc aat ggg aaa cta caa gat ggg tca atg gaa ata gac gct ggg aac   289
Ile Asn Gly Lys Leu Gln Asp Gly Ser Met Glu Ile Asp Ala Gly Asn
                85                  90                  95

aac ttg gag acc ttt aaa atg gga agt gga gct gaa gaa gca att gca 337
Asn Leu Glu Thr Phe Lys Met Gly Ser Gly Ala Glu Glu Ala Ile Ala
            100                 105                 110
gtt aat gat ttc cag aat ggc atc aca gga att cgt ttt gct gga gga 385
Val Asn Asp Phe Gln Asn Gly Ile Thr Gly Ile Arg Phe Ala Gly Gly
        115                 120                 125
gag aag tgc tac att aaa gcg caa gtg aag gct cgt att cct gag gtg 433
Glu Lys Cys Tyr Ile Lys Ala Gln Val Lys Ala Arg Ile Pro Glu Val
    130                 135                 140
ggc gcc gtg acc aaa cag agc atc tcc tcc aaa ctg gaa ggc aag atc 481
Gly Ala Val Thr Lys Gln Ser Ile Ser Ser Lys Leu Glu Gly Lys Ile
145                 150                 155                 160
atg cca gtc aaa tat gaa gaa aat tct ctt atc tgg gtg gct gta gat 529
Met Pro Val Lys Tyr Glu Glu Asn Ser Leu Ile Trp Val Ala Val Asp
                165                 170                 175
cag cct gtg aag gac aac agc ttc ttg agt tct aag gtg tta gaa ctc 577
Gln Pro Val Lys Asp Asn Ser Phe Leu Ser Ser Lys Val Leu Glu Leu
            180                 185                 190
tgc ggt gac ctt cct att ttc tgg ctt aaa cca acc tat cca aaa gaa 625
Cys Gly Asp Leu Pro Ile Phe Trp Leu Lys Pro Thr Tyr Pro Lys Glu
        195                 200                 205
atc cag agg gaa aga aga gaa gtg gta aga aaa att gtt cca act acc 673
Ile Gln Arg Glu Arg Arg Glu Val Val Arg Lys Ile Val Pro Thr Thr
    210                 215                 220
aca aaa aga cca cac agt gga cca cgg agc aac cca ggc gct gga aga 721
Thr Lys Arg Pro His Ser Gly Pro Arg Ser Asn Pro Gly Ala Gly Arg
225                 230                 235                 240
ctg aat aat gaa acc aga ccc agt gtt caa gag gac tca caa gcc ttc 769
Leu Asn Asn Glu Thr Arg Pro Ser Val Gln Glu Asp Ser Gln Ala Phe
                245                 250                 255
aat cct gat aat cct tat cat cag cag gaa ggg gaa agc atg aca ttc 817
Asn Pro Asp Asn Pro Tyr His Gln Gln Glu Gly Glu Ser Met Thr Phe
            260                 265                 270
gac cct aga ctg gat cac gaa gga atc tgt tgt ata gaa tgt agg cgg 865
Asp Pro Arg Leu Asp His Glu Gly Ile Cys Cys Ile Glu Cys Arg Arg
        275                 280                 285
agc tac acc cac tgc cag aag atc tgt gaa ccc ctg ggg ggc tat tac 913
Ser Tyr Thr His Cys Gln Lys Ile Cys Glu Pro Leu Gly Gly Tyr Tyr
    290                 295                 300
cca tgg cct tat aat tat caa ggc tgc cgt tcg gcc tgc aga gtc atc 961
Pro Trp Pro Tyr Asn Tyr Gln Gly Cys Arg Ser Ala Cys Arg Val Ile
305                 310                 315                 320
atg cca tgt agc tgg tgg gtg gcc cgc atc ctg ggc atg gtg taa 1006

Met Pro Cys Ser Trp Trp Val Ala Arg Ile Leu Gly Met Val
                325                 330

<210> 2
<211> 334
<212> PRT
<213> Homo sapiens

<400> 2
Met Thr Glu Asn Ser Asp Lys Val Pro Ile Ala Leu Val Gly Pro Asp
1               5                   10                  15
Asp Val Glu Phe Cys Ser Pro Pro Ala Tyr Ala Thr Leu Thr Val Lys
            20                  25                  30
Pro Ser Ser Pro Ala Arg Leu Leu Lys Val Gly Ala Val Val Leu Ile
        35                  40                  45
Ser Gly Ala Val Leu Leu Leu Phe Gly Ala Ile Gly Ala Phe Tyr Phe
    50                  55                  60
Trp Lys Gly Ser Asp Ser His Ile Tyr Asn Val His Tyr Thr Met Ser
65                  70                  75                  80
Ile Asn Gly Lys Leu Gln Asp Gly Ser Met Glu Ile Asp Ala Gly Asn
                85                  90                  95
Asn Leu Glu Thr Phe Lys Met Gly Ser Gly Ala Glu Glu Ala Ile Ala
            100                 105                 110
Val Asn Asp Phe Gln Asn Gly Ile Thr Gly Ile Arg Phe Ala Gly Gly
        115                 120                 125
Glu Lys Cys Tyr Ile Lys Ala Gln Val Lys Ala Arg Ile Pro Glu Val
    130                 135                 140
Gly Ala Val Thr Lys Gln Ser Ile Ser Ser Lys Leu Glu Gly Lys Ile
145                 150                 155                 160
Met Pro Val Lys Tyr Glu Glu Asn Ser Leu Ile Trp Val Ala Val Asp
                165                 170                 175
Gln Pro Val Lys Asp Asn Ser Phe Leu Ser Ser Lys Val Leu Glu Leu
            180                 185                 190
Cys Gly Asp Leu Pro Ile Phe Trp Leu Lys Pro Thr Tyr Pro Lys Glu
        195                 200                 205
Ile Gln Arg Glu Arg Arg Glu Val Val Arg Lys Ile Val Pro Thr Thr
    210                 215                 220
Thr Lys Arg Pro His Ser Gly Pro Arg Ser Asn Pro Gly Ala Gly Arg
225                 230                 235                 240
Leu Asn Asn Glu Thr Arg Pro Ser Val Gln Glu Asp Ser Gln Ala Phe
                245                 250                 255
Asn Pro Asp Asn Pro Tyr His Gln Gln Glu Gly Glu Ser Met Thr Phe
            260                 265                 270

Asp Pro Arg Leu Asp His Glu Gly Ile Cys Cys Ile Glu Cys Arg Arg
        275                 280                 285
Ser Tyr Thr His Cys Gln Lys Ile Cys Glu Pro Leu Gly Gly Tyr Tyr
    290                 295                 300
Pro Trp Pro Tyr Asn Tyr Gln Gly Cys Arg Ser Ala Cys Arg Val Ile
305                 310                 315                 320
Met Pro Cys Ser Trp Trp Val Ala Arg Ile Leu Gly Met Val
                325                 330

<210> 3
<211> 1006
<212> DNA
<213> Homo sapiens

<220>
<221> CDS
<222> (2)..(1003)

<400> 3
c atg aca gag aac tcc gac aaa gtt ccc att gcc ctg gtg gga cct gat 49
  Met Thr Glu Asn Ser Asp Lys Val Pro Ile Ala Leu Val Gly Pro Asp
  1               5                   10                  15
gac gtg gaa ttc tgc agc ccc ccg gcg tac gct acg ctg acg gtg aag    97
Asp Val Glu Phe Cys Ser Pro Pro Ala Tyr Ala Thr Leu Thr Val Lys
            20                  25                  30
ccc tcc agc ccc gcg cgg ctg ctc aag gtg gga gcc gtg gtc ctc att    145
Pro Ser Ser Pro Ala Arg Leu Leu Lys Val Gly Ala Val Val Leu Ile
        35                  40                  45
tcg gga gct gtg ctg ctg ctc ttt ggg gcc atc ggg gcc ttc tac ttc    193
Ser Gly Ala Val Leu Leu Leu Phe Gly Ala Ile Gly Ala Phe Tyr Phe
    50                  55                  60
tgg aag ggg agc gac agt cac att tac aat gtc cat tac acc atg agt    241
Trp Lys Gly Ser Asp Ser His Ile Tyr Asn Val His Tyr Thr Met Ser
65                  70                  75                  80
atc aat ggg aaa cta caa gat ggg tca atg gaa ata gac gct ggg aac    289
Ile Asn Gly Lys Leu Gln Asp Gly Ser Met Glu Ile Asp Ala Gly Asn
                85                  90                  95
aac ttg gag acc ttt aaa atg gga agt gga gct gaa gaa gca att gca    337
Asn Leu Glu Thr Phe Lys Met Gly Ser Gly Ala Glu Glu Ala Ile Ala
            100                 105                 110
gtt aat gat ttc cag aat ggc atc aca gga att cgt ttt gct gga gga    385
Val Asn Asp Phe Gln Asn Gly Ile Thr Gly Ile Arg Phe Ala Gly Gly
        115                 120                 125

gag aag tgc tac att aaa gcg caa gtg aag gct cgt att cct gag gtg 433
Glu Lys Cys Tyr Ile Lys Ala Gln Val Lys Ala Arg Ile Pro Glu Val
    130                 135                 140
ggc gcc gtg acc aaa cag agc atc tcc tcc aaa ctg gaa ggc aag atc 481
Gly Ala Val Thr Lys Gln Ser Ile Ser Ser Lys Leu Glu Gly Lys Ile
145                 150                 155                 160
atg cca gtc aaa tat gaa gaa aat tct ctt atc tgg gtg gct gta gat 529
Met Pro Val Lys Tyr Glu Glu Asn Ser Leu Ile Trp Val Ala Val Asp
                165                 170                 175
cag cct gtg aag gac aac agc ttc ttg aat tct aag gtg tta gaa ctc 577
Gln Pro Val Lys Asp Asn Ser Phe Leu Asn Ser Lys Val Leu Glu Leu
            180                 185                 190
tgc ggt gac ctt cct att ttc tgg ctt aaa cca acc tat cca aaa gaa 625
Cys Gly Asp Leu Pro Ile Phe Trp Leu Lys Pro Thr Tyr Pro Lys Glu
        195                 200                 205
atc cag agg gaa aga aga gaa gtg gta aga aaa att gtt cca act acc 673
Ile Gln Arg Glu Arg Arg Glu Val Val Arg Lys Ile Val Pro Thr Thr
    210                 215                 220
aca aaa aga cca cac agt gga cca cgg agc aac cca ggc gct gga aga 721
Thr Lys Arg Pro His Ser Gly Pro Arg Ser Asn Pro Gly Ala Gly Arg
225                 230                 235                 240
ctg aat aat gaa acc aga ccc agt gtt caa gag gac tca caa gcc ttc 769
Leu Asn Asn Glu Thr Arg Pro Ser Val Gln Glu Asp Ser Gln Ala Phe
                245                 250                 255
aat cct gat aat cct tat cat cag cag gaa ggg gaa agc atg aca ttc 817
Asn Pro Asp Asn Pro Tyr His Gln Gln Glu Gly Glu Ser Met Thr Phe
            260                 265                 270
gac cct aga ctg gat cac gaa gga atc tgt tgt ata gaa tgt agg cgg 865
Asp Pro Arg Leu Asp His Glu Gly Ile Cys Cys Ile Glu Cys Arg Arg
        275                 280                 285
agc tac acc cac tgc cag aag atc tgt gaa ccc ctg ggg ggc tat tac 913
Ser Tyr Thr His Cys Gln Lys Ile Cys Glu Pro Leu Gly Gly Tyr Tyr
    290                 295                 300
cca tgg cct tat aat tat caa ggc tgc cgt tcg gcc tgc aga gtc atc 961
Pro Trp Pro Tyr Asn Tyr Gln Gly Cys Arg Ser Ala Cys Arg Val Ile
305                 310                 315                 320
atg cca tgt agc tgg tgg gtg gcc cgc atc ctg ggc atg gtg taa 1006
Met Pro Cys Ser Trp Trp Val Ala Arg Ile Leu Gly Met Val
                325                 330

<210> 4
<211> 334
<212> PRT

<213> Homo sapiens

<400> 4
Met Thr Glu Asn Ser Asp Lys Val Pro Ile Ala Leu Val Gly Pro Asp
1               5                   10                  15
Asp Val Glu Phe Cys Ser Pro Pro Ala Tyr Ala Thr Leu Thr Val Lys
            20                  25                  30
Pro Ser Ser Pro Ala Arg Leu Leu Lys Val Gly Ala Val Val Leu Ile
        35                  40                  45
Ser Gly Ala Val Leu Leu Leu Phe Gly Ala Ile Gly Ala Phe Tyr Phe
    50                  55                  60
Trp Lys Gly Ser Asp Ser His Ile Tyr Asn Val His Tyr Thr Met Ser
65                  70                  75                  80
Ile Asn Gly Lys Leu Gln Asp Gly Ser Met Glu Ile Asp Ala Gly Asn
                85                  90                  95
Asn Leu Glu Thr Phe Lys Met Gly Ser Gly Ala Glu Glu Ala Ile Ala
            100                 105                 110
Val Asn Asp Phe Gln Asn Gly Ile Thr Gly Ile Arg Phe Ala Gly Gly
        115                 120                 125
Glu Lys Cys Tyr Ile Lys Ala Gln Val Lys Ala Arg Ile Pro Glu Val
    130                 135                 140
Gly Ala Val Thr Lys Gln Ser Ile Ser Ser Lys Leu Glu Gly Lys Ile
145                 150                 155                 160
Met Pro Val Lys Tyr Glu Glu Asn Ser Leu Ile Trp Val Ala Val Asp
                165                 170                 175
Gln Pro Val Lys Asp Asn Ser Phe Leu Asn Ser Lys Val Leu Glu Leu
            180                 185                 190
Cys Gly Asp Leu Pro Ile Phe Trp Leu Lys Pro Thr Tyr Pro Lys Glu
        195                 200                 205
Ile Gln Arg Glu Arg Arg Glu Val Val Arg Lys Ile Val Pro Thr Thr
    210                 215                 220
Thr Lys Arg Pro His Ser Gly Pro Arg Ser Asn Pro Gly Ala Gly Arg
225                 230                 235                 240
Leu Asn Asn Glu Thr Arg Pro Ser Val Gln Glu Asp Ser Gln Ala Phe
                245                 250                 255
Asn Pro Asp Asn Pro Tyr His Gln Gln Glu Gly Glu Ser Met Thr Phe
            260                 265                 270
Asp Pro Arg Leu Asp His Glu Gly Ile Cys Cys Ile Glu Cys Arg Arg
        275                 280                 285
Ser Tyr Thr His Cys Gln Lys Ile Cys Glu Pro Leu Gly Gly Tyr Tyr
    290                 295                 300
Pro Trp Pro Tyr Asn Tyr Gln Gly Cys Arg Ser Ala Cys Arg Val Ile
305                 310                 315                 320

Met Pro Cys Ser Trp Trp Val Ala Arg Ile Leu Gly Met Val
                325                 330

<210> 5
<211> 892
<212> DNA
<213> Homo sapiens

<220>
<221> CDS
<222> (2)..(889)

<400> 5
c atg aca gag aac tcc gac aaa gtt ccc att gcc ctg gtg gga cct gat 49
  Met Thr Glu Asn Ser Asp Lys Val Pro Ile Ala Leu Val Gly Pro Asp
  1               5                   10                  15
gac gtg gaa ttc tgc agc ccc ccg gcg tac gct acg ctg acg gtg aag    97
Asp Val Glu Phe Cys Ser Pro Pro Ala Tyr Ala Thr Leu Thr Val Lys
            20                  25                  30
ccc tcc agc ccc gcg cgg ctg ctc aag gtg gga gcc gtg gtc ctc att    145
Pro Ser Ser Pro Ala Arg Leu Leu Lys Val Gly Ala Val Val Leu Ile
        35                  40                  45
tcg gga gct gtg ctg ctg ctc ttt ggg gcc atc ggg gcc ttc tac ttc    193
Ser Gly Ala Val Leu Leu Leu Phe Gly Ala Ile Gly Ala Phe Tyr Phe
    50                  55                  60
tgg aag ggg agc gac agt cac att tac aat gtc cat tac acc atg agt    241
Trp Lys Gly Ser Asp Ser His Ile Tyr Asn Val His Tyr Thr Met Ser
65                  70                  75                  80
atc aat ggg aaa tta caa gat ggg tca atg gaa ata gac gct ggg aac    289
Ile Asn Gly Lys Leu Gln Asp Gly Ser Met Glu Ile Asp Ala Gly Asn
                85                  90                  95
aac ttg gag acc ttt aaa atg gga agt gga gct gaa gaa gca att gca    337
Asn Leu Glu Thr Phe Lys Met Gly Ser Gly Ala Glu Glu Ala Ile Ala
            100                 105                 110
gtt aat gat ttc cag aat gaa ggc aag atc atg cca gtc aaa tat gaa    385
Val Asn Asp Phe Gln Asn Glu Gly Lys Ile Met Pro Val Lys Tyr Glu
        115                 120                 125
gaa aat tct ctt atc tgg gtg gct gta gat cag cct gtg aag gac aac    433
Glu Asn Ser Leu Ile Trp Val Ala Val Asp Gln Pro Val Lys Asp Asn
    130                 135                 140
agc ttc ttg agt tct aag gtg tta gaa ctc tgc ggt gac ctt cct att    481
Ser Phe Leu Ser Ser Lys Val Leu Glu Leu Cys Gly Asp Leu Pro Ile
145                 150                 155                 160

tcc tgg ctt aaa cca acc tat cca aaa gaa atc cag agg gaa aga aga    529
Ser Trp Leu Lys Pro Thr Tyr Pro Lys Glu Ile Gln Arg Glu Arg Arg
                165                 170                 175
gaa gtg gta aga aaa att gtt cca act acc aca aaa aga cca cac aat    577
Glu Val Val Arg Lys Ile Val Pro Thr Thr Thr Lys Arg Pro His Asn
            180                 185                 190
gga cca cgg agc aac cca ggc gct gga aga ctg aat aat gaa acc aga    625
Gly Pro Arg Ser Asn Pro Gly Ala Gly Arg Leu Asn Asn Glu Thr Arg
        195                 200                 205
ccc agt gtt caa gag gac tca caa gcc ttc aat cct gat aat cct tat    673
Pro Ser Val Gln Glu Asp Ser Gln Ala Phe Asn Pro Asp Asn Pro Tyr
    210                 215                 220
cat cag cag gaa ggg gaa agc atg aca ttc gac cct aga ctg gat cac    721
His Gln Gln Glu Gly Glu Ser Met Thr Phe Asp Pro Arg Leu Asp His
225                 230                 235                 240
gaa gga atc tgt tgt ata gaa tgt agg cgg agc tac acc cac tgc cag    769
Glu Gly Ile Cys Cys Ile Glu Cys Arg Arg Ser Tyr Thr His Cys Gln
                245                 250                 255
aag atc tgt gaa ccc ctg ggg ggc tat tac cca tgg cct tat aat tat    817
Lys Ile Cys Glu Pro Leu Gly Gly Tyr Tyr Pro Trp Pro Tyr Asn Tyr
            260                 265                 270
caa ggc tgc cgt tcg gcc tgc aga gtc atc atg cca tgt agc tgg tgg    865
Gln Gly Cys Arg Ser Ala Cys Arg Val Ile Met Pro Cys Ser Trp Trp
        275                 280                 285
gtg gcc cgc atc ctg ggc atg gtg taa                                892
Val Ala Arg Ile Leu Gly Met Val
    290                 295

<210> 6
<211> 296
<212> PRT
<213> Homo sapiens

<400> 6
Met Thr Glu Asn Ser Asp Lys Val Pro Ile Ala Leu Val Gly Pro Asp
1               5                   10                  15
Asp Val Glu Phe Cys Ser Pro Pro Ala Tyr Ala Thr Leu Thr Val Lys
            20                  25                  30
Pro Ser Ser Pro Ala Arg Leu Leu Lys Val Gly Ala Val Val Leu Ile
        35                  40                  45
Ser Gly Ala Val Leu Leu Leu Phe Gly Ala Ile Gly Ala Phe Tyr Phe
    50                  55                  60
Trp Lys Gly Ser Asp Ser His Ile Tyr Asn Val His Tyr Thr Met Ser

65                  70                  75                  80
Ile Asn Gly Lys Leu Gln Asp Gly Ser Met Glu Ile Asp Ala Gly Asn
                85                  90                  95
Asn Leu Glu Thr Phe Lys Met Gly Ser Gly Ala Glu Glu Ala Ile Ala
            100                 105                 110
Val Asn Asp Phe Gln Asn Glu Gly Lys Ile Met Pro Val Lys Tyr Glu
        115                 120                 125
Glu Asn Ser Leu Ile Trp Val Ala Val Asp Gln Pro Val Lys Asp Asn
    130                 135                 140
Ser Phe Leu Ser Ser Lys Val Leu Glu Leu Cys Gly Asp Leu Pro Ile
145                 150                 155                 160
Ser Trp Leu Lys Pro Thr Tyr Pro Lys Glu Ile Gln Arg Glu Arg Arg
                165                 170                 175
Glu Val Val Arg Lys Ile Val Pro Thr Thr Thr Lys Arg Pro His Asn
            180                 185                 190
Gly Pro Arg Ser Asn Pro Gly Ala Gly Arg Leu Asn Asn Glu Thr Arg
        195                 200                 205
Pro Ser Val Gln Glu Asp Ser Gln Ala Phe Asn Pro Asp Asn Pro Tyr
    210                 215                 220
His Gln Gln Glu Gly Glu Ser Met Thr Phe Asp Pro Arg Leu Asp His
225                 230                 235                 240
Glu Gly Ile Cys Cys Ile Glu Cys Arg Arg Ser Tyr Thr His Cys Gln
                245                 250                 255
Lys Ile Cys Glu Pro Leu Gly Gly Tyr Tyr Pro Trp Pro Tyr Asn Tyr
            260                 265                 270
Gln Gly Cys Arg Ser Ala Cys Arg Val Ile Met Pro Cys Ser Trp Trp
        275                 280                 285
Val Ala Arg Ile Leu Gly Met Val
    290                 295

## Description

### Technical Field

The present invention relates to a drug containing, as an active ingredient, a chondromodulin-I protein (also referred to as "ChM-I" or "chondromodulin-I" protein hereinafter), which exhibits an activity of growing articular chondrocytes by itself or under coexistence of a basic fibroblast growth factor and an activity of suppressing hyper degradation of cartilage matrix. In particular, the present invention relates to a drug effective for therapeutic treatments of osteoarthritis and chronic rheumatoid arthritis.

### Background Art

Osteoarthritis (also referred to as "OA" hereinafter) and chronic rheumatoid arthritis (also referred to as "RA" hereinafter) are known as typical diseases that cause deteriorations of joint structure and function. The former is considered to be closely associated with aging or external injuries, and the latter is considered to be closely associated with abnormality in immune responses. Although fundamental etiologies of these pathology are different, hyper degradation of cartilage matrix caused by various proteases such as matrix metalloprotease produced by synovial membrane cells or cartilage cells themselves is commonly involved in the process of degeneration of the joint structure, in particular, cartilage tissue. This process can ultimately lead to loss of chondrocytes and even defect of cartilage tissues. Conventionally, for OA; analgestics or anti-inflammatory drugs are administered for the purpose of pain relief, or hyaluronic acid preparations are intraarticularly administered for the purpose of improvement of joint lubrication as symptomatic therapies. For RA, control of immunopathy using immunomodulators constitutes the mainline of the therapy. However, at present, there is no drug effective for the purposes of positively preventing the progression of joint destruction attributable to the degeneration of bones and cartilage tissues, which is an essential part of the pathological conditions, or regenerating the bones and cartilage tissues.

Further, a chondrocyte growth agent containing a hChM (human chondromodulin) I protein is described in Japanese Patent Laid-open Publication (Kokai) No. 7-138295. This publication discloses the nucleotide sequence of DNA coding for the hChM-I protein and a method for producing the hChM-I protein using that DNA.

This publication describes that the human chondromodulin-I protein has an activity of growing costal chondrocytes and an activity of suppressing growth of vascular endothelial cells. However, it is not known that the human chondromodulin-I protein has an activity of growing articular chondrocytes by itself or under coexistence of a basic fibroblast growth factor and an activity of suppressing hyper degradation of cartilage matrix. Further, there has so far been no report on an attempt of utilizing these activities for therapeutic agents for osteoarthritis and therapeutic agents for chronic rheumatoid arthritis.

### Disclosure of the Invention

The present invention has been accomplished in view of the above. An object of the present invention is to provide a drug exhibiting an activity of growing articular chondrocytes and an activity of suppressing hyper degradation of cartilage matrix, which can be effectively used as an articular chondrocyte growth agent, an agent for suppressing hyper degradation of cartilage matrix, or a therapeutic agent for various diseases such as diseases caused by suppression of the growth of articular chondrocytes and diseases caused by hyper degradation of cartilage matrix, in particular, osteoarthritis and chronic rheumatoid arthritis.

The inventors of the present invention assiduously studied in order to achieve the foregoing object. As a result, they found that a chondromodulin-I protein had an activity of growing articular chondrocytes by itself or under coexistence of a basic fibroblast growth factor (also referred to as "chondrocyte growth promoting activity" hereinafter) and an activity of suppressing hyper degradation of cartilage matrix (also referred to as "cartilage matrix hyper degradation suppressing activity" hereinafter), and thus accomplished the present invention.

That is, the present invention provides the followings.
(1) A therapeutic agent for osteoarthritis, which contains a chondromodulin-I protein having activities of the following (i) and (ii) as an active ingredient:
   (i) an activity of growing articular chondrocytes by itself or under coexistence of a basic fibroblast growth factor;
   (ii) an activity of suppressing hyper degradation of cartilage matrix.
(2) A therapeutic agent for osteoarthritis, which contains a protein defined in the following (a) or (b) as an active ingredient:
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(3) The therapeutic agent for osteoarthritis according to (1) or (2), which further contains a basic fibroblast growth factor.
(4) A therapeutic agent for osteoarthritis, which contains DNA coding for a protein defined in the following (a) or (b) :
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(5) The therapeutic agent for osteoarthritis according to (4), wherein the DNA is DNA defined in the following (c) or (d):
   (c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
   (d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
      (i) an activity of growing articular chondrocytes by itself or under coexistence of a basic fibroblast growth factor;
      (ii) an activity of suppressing hyper degradation of cartilage matrix.
(6) The therapeutic agent for osteoarthritis according to (4) or (5), which is a drug for gene therapy containing a vector that contains the DNA mentioned in (4) or (5) and can be expressed in an animal.
(7) A therapeutic agent for chronic rheumatoid arthritis, which contains a chondromodulin-I protein having activities of the following (i) and (ii) as an active ingredient:
   (i) an activity of growing articular chondrocytes by itself or under coexistence of a basic fibroblast growth factor;
   (ii) an activity of suppressing hyper degradation of cartilage matrix.
(8) A therapeutic agent for chronic rheumatoid arthritis, which contains a protein defined in the following (a) or (b) as an active ingredient:
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(9) The therapeutic agent for chronic rheumatoid arthritis according to (7) or (8), which further contains a basic fibroblast growth factor.
(10) A therapeutic agent for chronic rheumatoid arthritis, which contains DNA coding for a protein defined in the following (a) or (b):
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(11) The therapeutic agent for chronic rheumatoid arthritis according to (10), wherein the DNA is DNA defined in the following (c) or (d):
   (c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
   (d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
      (i) an activity of growing articular chondrocytes by itself or under coexistence of a basic fibroblast growth factor;
      (ii) an activity of suppressing hyper degradation of cartilage matrix.
(12) The therapeutic agent for chronic rheumatoid arthritis according to (10) or (11), which is a drug for gene therapy containing a vector that contains the DNA mentioned in (10) or (11) and can be expressed in an animal.
(13) An articular chondrocyte growth agent, which contains a chondromodulin-I protein having activities of the following (i) and (ii) as an active ingredient:
   (i) an activity of growing articular chondrocytes by itself or under coexistence of a basic fibroblast growth factor;
   (ii) an activity of suppressing hyper degradation of cartilage matrix.
(14) An articular chondrocyte growth agent, which contains a protein defined in the following (a) or (b) as an active ingredient:
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(15) The articular chondrocyte growth agent according to (13) or (14), which further contains a basic fibroblast growth factor.
(16) An articular chondrocyte growth agent, which contains DNA coding for a protein defined in the following (a) or (b):
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii).:
      (i) an activity of growing articular chondrocytes by itself or under coexistence of a basic fibroblast growth factor;
      (ii) an activity of suppressing hyper degradation of cartilage matrix.
(17) The articular chondrocyte growth agent according to (16), wherein the DNA is DNA defined in the following (c) or (d) :
   (c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
   (d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
      (i) an activity of growing articular chondrocytes by itself or under coexistence of a basic fibroblast growth factor;
      (ii) an activity of suppressing hyper degradation of cartilage matrix.
(18) The articular chondrocyte growth agent according to (16) or (17), which is a drug for gene therapy containing a vector that contains the DNA mentioned in (16) or (17) and can be expressed in an animal.
(19) An agent for suppressing hyper degradation of cartilage matrix, which contains a chondromodulin-I protein having activities of the following (i) and (ii) as an active ingredient:
   (i) an activity of growing articular chondrocytes by itself or under coexistence of a basic fibroblast growth factor;
   (ii) an activity of suppressing hyper degradation of cartilage matrix.
(20) An agent for suppressing hyper degradation of cartilage matrix, which contains a protein defined in the following (a) or (b) as an active ingredient:
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(21) The agent for suppressing hyper degradation of cartilage matrix according to (19) or (20), which further contains a basic fibroblast growth factor.
(22) An agent for suppressing hyper degradation of cartilage matrix, which contains DNA coding for a protein defined in the following (a) or (b):
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(23) The agent for suppressing hyper degradation of cartilage matrix according to (22), wherein the DNA is DNA defined in the following (c) or (d):
   (c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
   (d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
      (i) an activity of growing articular chondrocytes by itself or under coexistence of a basic fibroblast growth factor;
      (ii) an activity of suppressing hyper degradation of cartilage matrix.
(24) The agent for suppressing hyper degradation of cartilage matrix according to (22) or (23), which is a drug for gene therapy containing a vector that contains the DNA mentioned in (22) or (23) and can be expressed in an animal.
(25) A therapeutic agent for a disease caused by suppression of growth of articular chondrocytes, which contains a chondromodulin-I protein having activities of the following (i) and (ii) as an active ingredient.
   (i) an activity of growing articular chondrocytes by itself or under coexistence of a basic fibroblast growth factor;
   (ii) an activity of suppressing hyper degradation of cartilage matrix.
(26) A therapeutic agent for a disease caused by suppression of growth of articular chondrocytes, which contains a protein defined in the following (a) or (b) as an active ingredient:
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(27) The therapeutic agent for a disease caused by suppression of growth of articular chondrocytes according to (25) or (26), which further contains a basic fibroblast growth factor.
(28) A therapeutic agent for a disease caused by suppression of growth of articular chondrocytes, which contains DNA coding for a protein defined in the following (a) or (b):
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(29) The therapeutic agent for a disease caused by suppression of growth of articular chondrocytes according to (28), wherein the DNA is DNA defined in the following (c) or (d):
   (c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
   (d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii) :
      (i) an activity of growing articular chondrocytes by itself or under coexistence of a basic fibroblast growth factor;
      (ii) an activity of suppressing hyper degradation of cartilage matrix.
(30) The therapeutic agent for a disease caused by suppression of growth of articular chondrocytes according to (28) or (29), which is a drug for gene therapy containing a vector that contains the DNA mentioned in (28) or (29) and can be expressed in an animal.
(31) A therapeutic agent for a disease caused by hyper degradation of cartilage matrix, which contains a chondromodulin-I protein having activities of the following (i) and (ii) as an active ingredient:
   (i) an activity of growing articular chondrocytes by itself or under coexistence of a basic fibroblast growth factor;
   (ii) an activity of suppressing hyper degradation of cartilage matrix.
(32) A therapeutic agent for a disease caused by hyper degradation of cartilage matrix, which contains a protein defined in the following (a) or (b) as an active ingredient:
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(33) The therapeutic agent for a disease caused by hyper degradation of cartilage matrix according to (31) or (32), which further contains a basic fibroblast growth factor.
(34) A therapeutic agent for a disease caused by hyper degradation of cartilage matrix, which contains DNA coding for a protein defined in the following (a) or (b) :
   (a) a protein which has the amino acid sequence of SEQ ID NO: 2, 4 or 6;
   (b) a protein which has an amino acid sequence of SEQ ID NO: 2, 4 or 6 including deletion, substitution, insertion or addition of one or several amino acids, and has activities of the following (i) and (ii):
      (i) an activity of growing articular chondrocytes by itself or under coexistence of a basic fibroblast growth factor;
      (ii) an activity of suppressing hyper degradation of cartilage matrix.
(35) The therapeutic agent for a disease caused by hyper degradation of cartilage matrix according to (34), wherein the DNA is DNA defined in the following (c) or (d) :
   (c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
   (d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
      (i) an activity of growing articular chondrocytes by itself or under coexistence of a basic fibroblast growth factor;
      (ii) an activity of suppressing hyper degradation of cartilage matrix.
(36) The therapeutic agent for a disease caused by hyper degradation of cartilage matrix according'to (34) or (35), which is a drug for gene therapy containing a vector that contains the DNA mentioned in (34) or (35) and can be expressed in an animal.

Hereafter, the present invention will be explained in detail.

The drugs of the present invention used as articular chondrocyte growth agents, agents for suppressing hyper degradation of cartilage matrix, or therapeutic agents for various diseases including diseases caused by suppression of growth of articular chondrocytes or diseases caused by hyper degradation of cartilage matrix, in particular, diseases of osteoarthritis and chronic rheumatoid arthritis (also referred to as "drugs of the present invention" hereinafter) contain a chondromodulin-I protein as an active ingredient.

Chondromodulin-I used in the present invention is a protein having activities described in the following (i) and (ii):
(i) an activity of growing articular chondrocytes by itself or under coexistence of a basic fibroblast growth factor;
(ii) an activity of suppressing hyper degradation of cartilage matrix.

As chondromodulin-I having the aforementioned activities, human chondromodulin-I (hChM-I) can be mentioned.

hChM-I is a water-soluble protein composed of one polypeptide and has a molecular weight of about 26 kDa as measured by SDS-polyacrylamide gel electrophoresis. It has been reported that hChM-I has an activity of growing costal chondrocytes by itself or under coexistence of a fibroblast growth factor, an activity of promoting the differentiation function for costal chondrocytes and an activity of suppressing growth of vascular endothelial cells (Japanese Patent Laid-Open Publication (Kokai) No. 7-138295). The inventors of the present invention found that hChM-I had an activity of promoting growth of articular chondrocytes. Further, since hChM-I suppressed the production of neutral proteases (NP) including matrix metalloprotease-3 (MMP-3) derived from chondrocytes, they also found that hChM-I had an activity of suppressing hyper degradation of cartilage matrix.

Therefore, drugs containing a chondromodulin-I protein having the chondrocyte growth promoting activity and the cartilage matrix hyper degradation suppressing activity as an active ingredient can be used as articular chondrocyte growth agents, agents for suppressing hyper degradation of cartilage matrix, other therapeutic agents for diseases caused by suppression of growth of articular chondrocytes or therapeutic agents for diseases caused by hyper degradation of cartilage matrix. In particular, the aforementioned drugs can be preferably used as therapeutic agents for osteoarthritis and therapeutic agents for chronic rheumatoid arthritis among the diseases caused by suppression of growth of articular chondrocytes and diseases caused by hyper degradation of cartilage matrix. Further, the present invention also provides use of a chondromodulin-I protein having an activity of growing articular chondrocytes and an activity of suppressing hyper degradation of cartilage matrix in manufacture of various drugs such as therapeutic agents for osteoarthritis and therapeutic agent for chronic rheumatoid arthritis and methods for treating various diseases such as osteoarthritis and chronic rheumatoid arthritis, which comprise administering a therapeutically effective amount of a chondromodulin-I protein having an activity of growing articular chondrocytes and an activity of suppressing hyper degradation of cartilage matrix to a patient who needs growth of articular chondrocytes and suppression of hyper degradation of cartilage matrix.

Specific examples of the hChM-I protein include proteins having the amino acid sequence of SEQ ID NO: 2, 4 or 6 mentioned in Sequence Listing.

ChM-I used in the present invention can be obtained by extraction and purification from cartilage or cultured cells containing ChM-I. However, in view of mass production, it is preferable to produce ChM-I by a recombinant DNA technique using DNA coding for ChM-I. DNA coding for the hChM-I protein has already been cloned, and its nucleotide sequence has been elucidated. Further, methods for producing hChM-I using this DNA are described in Japanese Patent Laid-open Publication (Kokai) Nos. 7-138295, 9-299088 and so forth, and hChM-I produced by these methods can be preferably used in the present invention.

Nucleotide sequences of DNAs coding for the proteins having the aforementioned amino acid sequence of SEQ ID NO: 2, 4 or 6 mentioned in Sequence Listing are exemplified as SEQ ID NOS: 1, 3 and 5. The nucleotide numbers 2 to 1003 in the nucleotide sequence of SEQ ID NO: 1, the nucleotide numbers 2 to 1003 in the nucleotide sequence of SEQ ID NO: 3 and the nucleotide numbers 2 to 889 in the nucleotide sequence of SEQ ID NO: 5 are the coding regions.

Since the amino acid sequences of the hChM-I proteins and the nucleotide sequences of DNAs coding therefor have already been elucidated, the aforementioned DNAs coding for the hChM-I proteins can be obtained from human chromosomal DNA or chromosome library by PCR using primers prepared based on those sequences.

As ChM-I contained in the drugs of the present invention, chondromodulin proteins derived from animals other than humans and corresponding to hChM-I can also be used in addition to hChM-I so long as they have an chondrocyte growth promoting activity and a cartilage matrix hyper degradation suppressing activity and can be suitably used for treatment of humans.

Further, hChM-I or chondromodulin proteins derived from animals other than humans may have an amino acid mutation due to SNP (single nucleotide polymorphism) or the like so long as they have a chondrocyte growth promoting activity and a cartilage matrix hyper degradation suppressing activity. Further, hChM-I other than the currently known hChM-I or homologues thereof from animals other than humans can also be used in the present invention so long as they have functions equivalent to those of the aforementioned hChM-I. Alternatively, analogues of natural hChM-I including deletion, substitution, insertion, addition or the like of amino acids may also be used.

In view of antigenicity or the like, it is preferable to use hChM-I or analogues thereof. As the analogues of hChM-I, proteins which have an amino acid sequence of SEQ ID NO: 2, 4 or 6 including deletion, substitution, insertion or addition of one or several amino acids may be used so long as they have a chondrocyte growth promoting activity and a cartilage matrix hyper degradation suppressing activity. In the present invention, the term "several" means a number of about 2 to 30, preferably about 2 to 20, particularly preferably 2 to 10.

Further, the aforementioned DNA coding for chondromodulin-I can be used for gene therapy of osteoarthritis, chronic rheumatoid arthritis, diseases caused by suppression of growth of articular chondrocytes, diseases caused by hyper degradation of cartilage matrix and so forth. Examples of such DNA coding for chondromodulin-I include DNA coding for the amino acid sequence of SEQ ID NO: 2, 4 or 6 or DNA coding for a protein which has an amino acid sequence including deletion, substitution insertion or addition of one or several amino acids in any of these amino acid sequences, and has the activities of (i) and (ii). Specifically, there can be mentioned DNA containing the nucleotide sequence of the nucleotide numbers 2 to 1003 in the nucleotide sequence of SEQ ID NO: 1, the nucleotide numbers 2 to 1003 in the nucleotide sequence of SEQ ID NO: 3 or the nucleotide numbers 2 to 889 in the nucleotide sequence of SEQ ID NO: 5. Further, DNA of the present invention may be DNA which is hybridizable with the nucleotide sequence of the nucleotide numbers 2 to 1003 in the nucleotide sequence of SEQ ID NO: 1, the nucleotide numbers 2 to 1003 in the nucleotide sequence of SEQ ID NO: 3 or the nucleotide numbers 2 to 889 in the nucleotide sequence of SEQ ID NO: 5 or a probe that can be prepared from any of these sequences under a stringent condition, and codes for a protein having a chondrocyte growth promoting activity and a cartilage matrix hyper degradation suppressing activity. DNA coding for chondromodulin-I including deletion, substitution, insertion or addition of amino acid residues can be obtained by a conventional mutation techniques such as a method of using a mutagenesis agent or site-directed mutagenesis. The site-directed mutagenesis techniques include various techniques (R. Higuchi et al., Recombinant PCR in "PCR Protocols: A Guide to Methods and Applications" p.177, Academic Press, 1990; Sambrook, Fritsch and Maniatis, "Molecular Cloning" Chapter 15 Site-directed Mutagenesis of Cloned DNA, Cold Spring Harbor Laboratory Press, 1989 etc.), and any technique may be used so long as it is a technique of site-specifically introducing a mutation.

In preparation of DNA coding for hChM-I and production of hChM-I by using recombinant DNA techniques, methods for preparation of chromosomal DNA, construction of chromosomal DNA library, hybridization, PCR, preparation of plasmid DNA, digestion and ligation of DNA, transformation etc. are described in Japanese Patent Laid-open Publication (Kokai) No. 7-138295. Further, a method for mass production of a hChM-I protein using a DNA fragment obtained by changing 3 nucleotides starting from adenine coding for translation initiation methionine in the upstream of the nucleotide sequence coding for hChM-I is described in Japanese Patent Laid-open Publication (Kokai) No. 9-299088.

Since the drugs containing a ChM-I protein and drugs containing DNA coding for the protein of the present invention have actions of growing articular chondrocytes, suppressing hyper degradation of cartilage matrix and so forth, they are effective as prophylactic agents or therapeutic agents for diseases caused by suppression of growth of articular chondrocytes and diseases caused by hyper degradation of cartilage matrix, in particular, diseases of osteoarthritis and chronic rheumatoid arthritis.

In osteoarthritis and chronic rheumatoid arthritis, articular cartilage tissues become fragile with degradation of extracellular matrices of chondrocytes such as type II collagen and proteoglycan due to various factors, and advanced joint destruction including loss of chondrocytes is observed as a terminal pathologic status. In the present invention, it was demonstrated by using articular cartilage tissues, which constitute major lesions of these joint diseases, as experimental materials that hChM-I promoted the growth of articular chondrocytes. Further, it was also demonstrated that hChM-I suppressed the induction of activities of neutral proteases from chondrocytes associated with pathological hyper degradation of extracellular matrices of chondrocytes, and in particular, suppressed the production of MMP-3, which is considered to play the main role among them. Therefore, drugs containing a ChM-I protein and drugs containing DNA coding for this protein of the present invention are expected to potently inhibit the clinical progression of pathology of OA- and RA- joint and achieve structural regeneration by improving the conditions from both aspects of abnormal metabolism of extracellular matrices of chondrocytes and control of growth of chondrocytes themselves.

As for the drugs containing ChM-I as an active ingredient, ChM-I per se may be used as a preparation of ChM-I. However, it can also be mixed with a pharmaceutically acceptable carrier and used as a pharmaceutical composition. In this case, the proportion of ChM-I as an active ingredient based on the carrier ingredient can vary in the range of 1 to 90% by weight. For example, ChM-I of the present invention can be mixed with, impregnated into or coated on biocompatible carriers such as collagen, atelocollagen, gelatin, hyaluronic acid, polyethylene glycol, polylactic acid, bone cement, hydroxyapatite, ceramics, carbon fiber and fibrin adhesive, and administered to a fracture site, a cartilage disease site etc. as a drug for external use.

Further, ChM-I of the present invention may be prepared into a dosage form of granule, subtilized granule, powder, tablet, hard capsule, soft capsule, syrup, emulsion, suspension, solution or the like, and orally administered, or prepared as an injection and intravenously, intramuscularly, locally or subcutaneously administered. It can also be used as a suppository. When a composition for oral, enteral or parenteral administration is prepared, organic or inorganic, solid or liquid carriers or diluents are used.

As excipients used for producing solid preparations, for example, lactose, sucrose, starch, talc, cellulose, dextrin, kaolin, calcium carbonate and so forth are used. Liquid preparations for oral administration, that is, emulsion, syrup, suspension, solution etc., contain generally used inactive diluents, for example, water, vegetable oil and so forth. In addition to the inactive diluents, these preparations may contain auxiliary agents, for example, moistening agents, suspending aids, sweeteners, flavoring agents, coloring agents, preservatives and so forth. Liquid preparations may be produced and contained in a capsule made of a substance that can be absorbed, such as gelatin. Examples of solvents or suspending agents used for the production of preparations for parenteral administration, that is, injection, suppository etc., include, for example, water, propylene glycol, polyethylene glycol, benzyl alcohol, ethyl oleate, lecithin and so forth. Examples of bases used for suppository include, for example, cacao butter, emulsified cacao butter, lauric fat, witepsol and so forth. These preparations can be appropriately produced in a conventional manner.

The clinical dose of ChM-I in the drugs of the present invention is appropriately determined depending on the dosage form, age, body weight, symptoms of the patient etc. However, when it is used as an oral agent or a drug for external use, 1 ng to 50 mg/day (1/10 thereof or less in the case of injection) in terms of the amount of ChM-I is generally desirable for adult. This dose may be administered once a day, or two to several times a day at suitable intervals or intermittently. When ChM-I is used as an injection, the aforementioned dose is preferably administered repetitively and intermittently. ChM-I used for medical purposes may be any of purified ChM-I, recombinant ChM-I, culture broth of transformant, isolated transformant, transformant treated product, immobilized transformant, crude enzyme solution, enzyme-treated product etc.

The drugs of the present invention may contain ingredients exhibiting effect of growing articular chondrocytes or effect of suppressing hyper degradation of cartilage matrix, in particular, therapeutic effect for osteoarthritis or therapeutic effect for chronic rheumatoid arthritis, other than ChM-I, in combination with ChM-I so long as the effect of ChM-I is not degraded.

Further, the drugs of the present invention may contain a basic fibroblast growth factor together with ChM-I.

Further, when DNA coding for chondromodulin-I is used for gene therapy, various techniques conventionally used for gene therapy can be employed. Specifically, a chondromodulin-I gene can be expressed in the body of patient by a method comprising transplanting a viral vector such as retrovirus vector, adenovirus vector, AAV vector or herpes virus vector or a DNA expression vector coding for a chondromodulin-I obtained by the membrane fusion liposome method or the like into bone marrow cells of a patient with osteoarthritis, chronic rheumatoid arthritis or the like, who need growth of articular chondrocytes or suppression of hyper degradation of cartilage matrix (also referred to as "patient with osteoarthritis, chronic rheumatoid arthritis or the like") by the method described in International Patent Unexamined Publication in Japanese (KOHYO) No. 9-501046 etc. or a similar method, a method of administering such a vector to a muscular tissue, vascular system, intestine, skin, lung or the like of a patient with osteoarthritis, chronic rheumatoid arthritis or the like by the method described in International Patent Unexamined Publication in Japanese (KOHYO) No. 9-505084 etc. or a similar method, a method of administering such a vector to cerebrospinal fluid of a patient with osteoarthritis, chronic rheumatoid arthritis or the like by the method described in International Patent Unexamined Publication in Japanese (KOHYO) No. 9-505561 etc. or a similar method, and so forth. Further, by introducing a chondromodulin-I gene into egg cells of a patient with osteoarthritis, chronic rheumatoid arthritis or the like by the aforementioned methods, diseases caused by suppression of growth of articular chondrocytes or hyper degradation of cartilage matrix such as osteoarthritis and chronic rheumatoid arthritis in offspring of the patient can be prophylactically prevented.

### Brief Description of the Drawings

Fig. 1 shows the [³H]thymidine uptake ability of articular chondrocytes stimulated with bFGF in the presence and absence of hChM-I.
Fig. 2 shows measurement results of the [³H]thymidine uptake ability of articular chondrocytes stimulated with hChM-I in the presence and absence of bFGF.
Fig. 3 shows changes in the number of articular chondrocytes cultured in the presence of hChM-I.
Fig. 4 shows changes in the number of articular chondrocytes cultured in the presence of bFGF and hChM-I.
Fig. 5 shows the NP activity increased by stimulation with IL-1β in the culture medium of articular chondrocytes and effect of hChM-I and bFGF thereon.
Fig. 6 shows the MMP-3 concentration increased by stimulation with IL-1β in the culture medium of articular chondrocytes and effect of hChM-I and bFGF thereon.

### Best Mode for Carrying out the Invention

Hereafter, the present invention will be more specifically explained with reference to the following example.

The instruments, reagents and articular chondrocytes used in the example are mentioned below.

### <Instruments and reagents used>

The plastic instruments for culture were purchased from Iwaki Glass. Phosphate-buffered saline (PBS(-) not containing Ca²⁺ or Mg²⁺) was purchased from Dainippon Pharmaceutical Co., Ltd. Mitsubishi RDF-CHO Formula culture medium (referred to as "TS-2" hereinafter), penicillin G-streptomycin solution and trypsin-EDTA solution were purchased from Life Technologies Oriental. Collagenase (type II), trypsin (Trypsin TRCK treated) and trypsin inhibitor (soybean trypsin inhibitor) were purchased from Worthington Biochemical. Azocoll substrate (< 5.0 mesh) was purchased from Calbiochem. Matrix metalloproteinase-3 (MMP-3), rabbits, ELISA system was purchased from Amersham Pharmacia. Interleukin-1β (IL-1β) and basic fibroblast growth factor (bFGF) were purchased from PEPROTECH. Tritium-labeled thymidine ([³H]thymidine) was purchased from ICN Biomedicals. MeltiLex A was purchased from WALLAC.

### <Isolation and preparation of articular chondrocytes>

The articular chondrocytes used for evaluation were obtained as follows. That is, cartilage tissues were collected from both of the right and left knee joints and the shoulder joints of male Japanese white rabbits (purchased from Japan Laboratory Animals Inc.) with a body weight of about 1.5 kg and shaken in 0.025% trypsin-0.265 M EDTA/TS-2 at 37°C for 30 minutes, and then the supernatant was removed. Subsequently, the tissue sections were washed 3 times with 10% rabbit serum/TS-2 and shaken in 0.3% collagenase/TS-2 at 37°C for 60 minutes, and then the supernatant was removed. The tissue sections were shredded with a surgical blade and shaken in 0.15% collagenase/10% rabbit serum/TS-2 at 37°C for 3 hours for further digestion.

The digested tissue solution obtained by the above procedure was filtered through a cell strainer (40 µm mesh) and then centrifuged at 2000 rpm at 4°C for 5 minutes to obtain articular chondrocytes. The cells were washed with TS-2 and resuspended in 10% rabbit serum/TS-2 at a density of 1.5 to 2 x 10⁵ cells/ml. The cell suspension were plated in 96-well plates in a volume of 100 µl/well and cultured at 37°C in the presence of air containing 5% CO₂. The TS-2 used above was added with 100 U/ml of penicillin G and 100 µg/ml of streptomycin before use. Further, the rabbit serum was prepared by centrifugation from the whole blood of the same rabbit as used for the isolation of articular chondrocytes and inactivated at 56°C for 30 minutes before use.

### Example 1

### <1> Confirmation of activity of growing articular chondrocytes

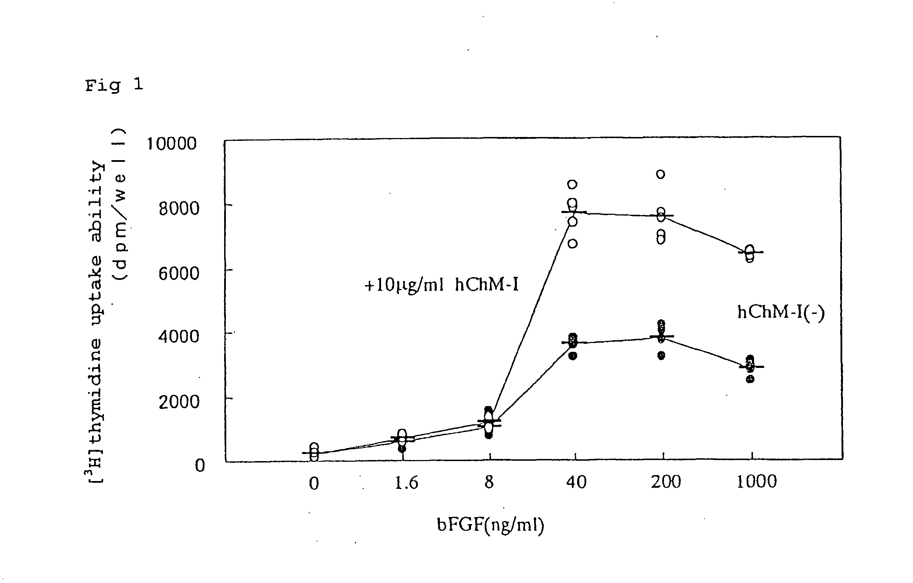

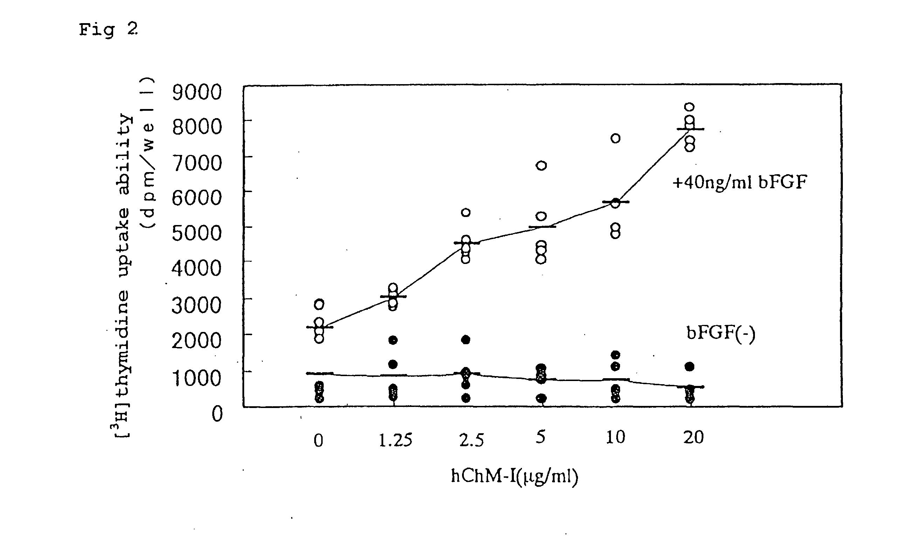

The effect of hChM-I on the growth of chondrocytes was evaluated in terms of changes in the DNA synthesis activity determined based on measurement of the [³H] thymidine uptake ability of articular chondrocytes and the number of cells. The [³H] thymidine uptake ability was measured as follows. The articular chondrocytes prepared by the above method were cultured until the cells reached a semi-confluent state, and then the culture medium was replaced with 100 µl/well of 0.5% rabbit serum/TS-2. The culture was continued for 24 hours, and then the culture medium was replaced with 100 µl/well of TS-2 containing various concentrations of bFGF (final concentrations: 0, 1.6, 8, 40, 200 and 1000 ng/ml) and hChM-I (final concentrations: 0, 1.25, 2.5, 5 and 10 µM). The culture was continued for 24 hours, then the culture was added with 10 µl/well of [³H]thymidine adjusted to 50 µCi/ml with PBS(-), and the culture was continued for further 5 hours. Each culture medium was removed by using a cell harvester (HARVESTER96 MACH IIIM, TOMTEC), then added with 100 µl/well of 0.025% trypsin-0.265 M EDTA/PBS(-) and incubated for 5 minutes. The floating cells were adsorbed on a glass filter (Printed Filter Mat A, WALLAC), and the filter was dried and then impregnated with a scintillator, MeltiLex A. The radioactivity was measured by using a β scintillation counter (TRILUX 145 MICROBETA, WALLAC). As a result, hChM-I used by itself hardly influenced the [³H]thymidine uptake ability, whereas it enhanced the [³H]thymidine uptake promotion by bFGF in a concentration-dependent manner (Figs. 1 and 2) .

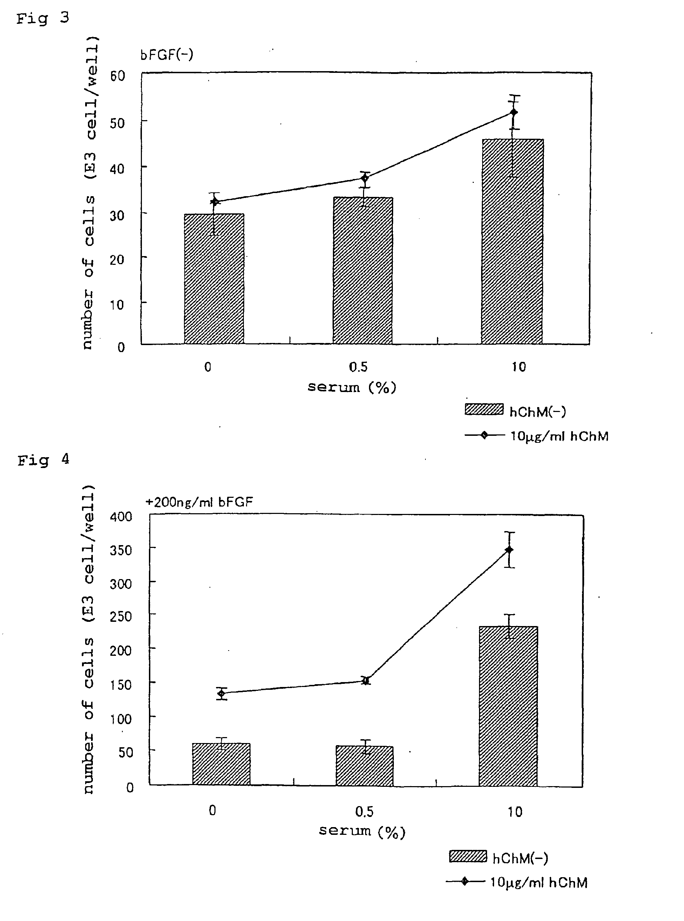

The changes in the number of cells were measured as follows. The culture medium of articular chondrocytes that reached a semiconfluent state was replaced with 100 µl/well of TS-2 containing various concentrations of the rabbit serum (final concentrations: 0, 0.5 and 10%), bFGF (final concentrations: 0 and 200 ng/ml) and hChM-I (final concentrations: 0 and 10 µM), and the culture was further continued for 3 days. Each culture medium was removed, and then the cells were detached and collected in 50 µl/well of 0.025% trypsin-0.265 M EDTA/PBS(-).
The number of cells was counted by using a hemacytometer (improved Neubauer ruling) under a microscope. As a result, hChM-I used by itself hardly influenced the number of articular chondrocytes or the increase in the number of cells with the increase of the serum concentration (Fig. 3). However, hChM-I enhanced the increase in the number of cells promoted by the addition of bFGF (Fig. 4). These results revealed that hChM-I promoted the growth of articular chondrocytes via an action for regulation of DNA synthesis cooperated with bFGF.

### <2> Confirmation of activity of suppressing hyper degradation of cartilage matrix (effect on increase in production of extracellular matrix-degradating enzymes in interleukin 1β-stimulated articular chondrocytes)

The effect of hChM-I on the production of chondrocyte extracellular matrix-degradating enzymes was evaluated in terms of the neutral protease (NP) activity and the MMP-3 concentration in a culture medium. That is, the articular chondrocytes prepared by the above method were cultured until the cells reached a confluent state, and then the culture medium was replaced with 100 µl/well of TS-2 containing various concentrations of IL-1β (final concentrations: 0 and 1 ng/ml), bFGF (final concentrations: 0 and 200 ng/ml) and hChM-I(final concentrations: 0, 2.5, 5, 10 and 20 µM). The culture was continued at 37°C for 24 hours in the presence of air containing 5% CO₂, and then the culture medium was collected and stored in a frozen state at -80°C until use in the measurement described later. Further, culture medium of rabbit articular chondrocytes separately treated with 1 ng/ml of IL-1β for 24 hours was prepared beforehand and stored as a standard.

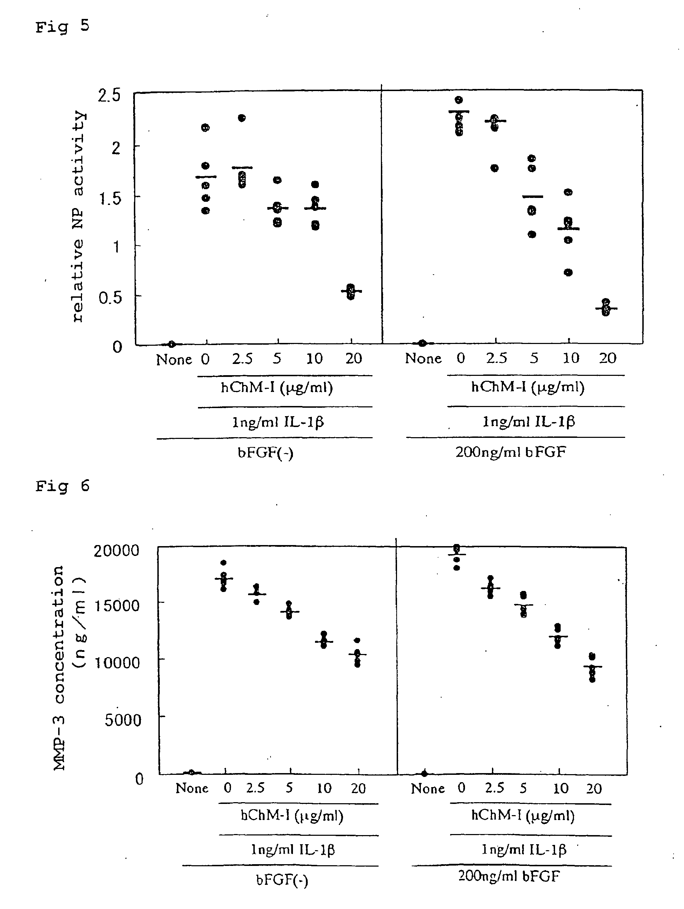

The NP activity was measured as follows. 75 µl of each sample and 75 µl of the standard serially diluted thawed at room temperature were added with 5 µl of 1 mg/ml trypsin solution and incubated at 37°C for 15 minutes to convert the inactive protease precursor in the sample into the active form. The mixture was further added with 5 µl of 5 mg/ml trypsin inhibitor solution to terminate the conversion reaction.
Subsequently, the Azocoll substrate suspension (60 µg/ml in 0.05 M tris(hydroxymethyl)aminomethane/hydrochloric acid buffer (pH 7.8) containing 1 mM calcium chloride, 125 µl/tube) placed into microtubes beforehand was added with 75 µl of the sample or the standard converted to the active form, sufficiently stirred and then incubated at 37°C for 24 hours. Undecomposed substrate and the supernatant containing dyes eluted along with the decomposition of the substrate were centrifuged at 3000 rpm for 10 minutes, and then 80 µl of each sample supernatant was immediately collected on a microplate. The absorbance of the supernatant was measured at 520 nm, the specific absorption wavelength of the dye, by using a microplate reader (SPECTRA MAX250, Molecular Devices). The NP activity of each sample was calculated as a relative activity from a regression line obtained by using the absorbance of the standard. As a result, hChM-I dose-dependently suppressed the NP activity of the culture medium of articular chondrocytes increased by the IL-1β stimulation irrespective of the presence or absence of bFGF (Fig. 5).

The MMP-3 concentration was measured by using a commercially available ELISA kit (Matrix Metalloproteinase-3 (MMP-3), rabbits, ELISA system) according to the attached instruction as follows. Each sample and a rabbit MMP-3 standard solution of a known concentration were added in a volume of 100 µl/well to a 96-well plate on which anti-rabbit MMP-3 monoclonal antibodies (specifically recognizing rabbit inactive MMP-3 precursor) were immobilized and incubated at 4°C for 2 hours. The samples were washed off, and then 100 µl/well of a peroxidase-labeled anti-rabbit MMP-3 monoclonal antibody solution was added to each well and incubated at 4°C for 1 hour. Unreacted antibodies were washed off, and 100 µl/well of a 3,3',5,5'-tetramethylbenzidine/hydrogen peroxide solution was added to each well and incubated at room temperature for 30 minutes. Immediately, the absorbance of the reaction mixture was measured at 450 nm, the specific absorption wavelength of the color of the reaction mixture developed with peroxidase, by using a microplate reader. The MMP-3 concentration in each sample was calculated from a regression line obtained by using the absorbance of the standard. As a result, hChM-I dose-dependently suppressed the increase of the MMP-3 concentration in the culture medium of articular chondrocytes promoted by the IL-1β stimulation irrespective of the presence or absence of bFGF (Fig. 6). The suppression of the increase in the NP activity by hChM-I shown in Fig. 5 can be at least partly explained based on the action for suppressing the synthesis of the MMP-3 enzyme protein in articular chondrocytes.

### Industrial Applicability

The present invention can provide articular chondrocyte growth agents, agents for suppressing hyper degradation of cartilage matrix and further, therapeutic agents that effectively act on various diseases including diseases caused by suppression of growth of articular chondrocytes and diseases caused by hyper degradation of cartilage matrix, in particular, osteoarthritis and chronic rheumatoid arthritis. Specifically, there can be provided various drugs such as therapeutic agents for osteoarthritis and therapeutic agents for chronic rheumatoid arthritis which contain a protein having an activity of growing articular chondrocytes and an activity of suppressing hyper degradation of cartilage matrix as an active ingredient.

## Claims

1. A therapeutic agent for osteoarthritis containing a chondromodulin-I protein having activities of the following (i) and (ii) as an active ingredient:
(i) an activity of growing articular chondrocytes by itself or under coexistence of a basic fibroblast growth factor;
(ii) an activity of suppressing hyper degradation of cartilage matrix.

2. A therapeutic agent for osteoarthritis, which contains a protein defined in the following (a) or (b) as an active ingredient:
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

3. The therapeutic agent for osteoarthritis according to claim 1 or 2, which further contains a basic fibroblast growth factor.

4. A therapeutic agent for osteoarthritis, which contains DNA coding for a protein defined in the following (a) or (b):
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

5. The therapeutic agent for osteoarthritis according to claim 4, wherein the DNA is DNA defined in the following (c) or (d):
(c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
(d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
(i) an activity of growing articular chondrocytes by itself or under coexistence of a basic fibroblast growth factor;
(ii) an activity of suppressing hyper degradation of cartilage matrix.

6. The therapeutic agent for osteoarthritis according to claim 4 or 5, which is a drug for gene therapy containing a vector that contains the DNA mentioned in claim 4 or 5 and can be expressed in an animal.

7. A therapeutic agent for chronic rheumatoid arthritis, which contains a chondromodulin-I protein having activities of the following (i) and (ii) as an active ingredient:
(i) an activity of growing articular chondrocytes by itself or under coexistence of a basic fibroblast growth factor;
(ii) an activity of suppressing hyper degradation of cartilage matrix.

8. A therapeutic agent for chronic rheumatoid arthritis, which contains a protein defined in the following (a) or (b) as an active ingredient:
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

9. The therapeutic agent for chronic rheumatoid arthritis according to claim 7 or 8, which further contains a basic fibroblast growth factor.

10. A therapeutic agent for chronic rheumatoid arthritis, which contains DNA coding for a protein defined in the following (a) or (b):
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

11. The therapeutic agent for chronic rheumatoid arthritis according to claim 10, wherein the DNA is DNA defined in the following (c) or (d):
(c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
(d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
(i) an activity of growing articular chondrocytes by itself or under coexistence of a basic fibroblast growth factor;
(ii) an activity of suppressing hyper degradation of cartilage matrix:

12. The therapeutic agent for chronic rheumatoid arthritis according to claim 10 or 11, which is a drug for gene therapy containing a vector that contains the DNA mentioned in claim 10 or 11 and can be expressed in an animal.

13. An articular chondrocyte growth agent, which contains a chondromodulin-I protein having activities of the following (i) and (ii) as an active ingredient:
(i) an activity of growing articular chondrocytes by itself or under coexistence of a basic fibroblast growth factor;
(ii) an activity of suppressing hyper degradation of cartilage matrix.

14. An articular chondrocyte growth agent, which contains a protein defined in the following (a) or (b) as an active ingredient:
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

15. The articular chondrocyte growth agent according to claim 13 or 14, which further contains a basic fibroblast growth factor.

16. An articular chondrocyte growth agent, which contains DNA coding for a protein defined in the following (a) or (b):
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

17. The articular chondrocyte growth agent according to claim 16, wherein the DNA is DNA defined in the following (c) or (d):
(c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
(d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
(i) an activity of growing articular chondrocytes by itself or under coexistence of a basic fibroblast growth factor;
(ii) an activity of suppressing hyper degradation of cartilage matrix.

18. The articular chondrocyte growth agent according to claim 16 or 17, which is a drug for gene therapy containing a vector that contains the DNA mentioned in claim 16 or 17 and can be expressed in an animal.

19. An agent for suppressing hyper degradation of cartilage matrix, which contains a chondromodulin-I protein having activities of the following (i) and (ii) as an active ingredient:
(i) an activity of growing articular chondrocytes by itself or under coexistence of a basic fibroblast growth factor;
(ii) an activity of suppressing hyper degradation of cartilage matrix.

20. An agent for suppressing hyper degradation of cartilage matrix, which contains a protein defined in the following (a) or (b) as an active ingredient:
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

21. The agent for suppressing hyper degradation of cartilage matrix according to claim 19 or 20, which further contains a basic fibroblast growth factor.

22. An agent for suppressing hyper degradation of cartilage matrix, which contains DNA coding for a protein defined in the following (a) or (b):
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

23. The agent for suppressing hyper degradation of cartilage matrix according to claim 22, wherein the DNA is DNA defined in the following (c) or (d):
(c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
(d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
(i) an activity of growing articular chondrocytes by itself or under coexistence of a basic fibroblast growth factor;
(ii) an activity of suppressing hyper degradation of cartilage matrix.

24. The agent for suppressing hyper degradation of cartilage matrix according to claim 22 or 23, which is a drug for gene therapy containing a vector that contains the DNA mentioned in claim 22 or 23 and can be expressed in an animal.

25. A therapeutic agent for a disease caused by suppression of growth of articular chondrocytes, which contains a chondromodulin-I protein having activities of the following (i) and (ii) as an active ingredient:
(i) an activity of growing articular chondrocytes by itself or under coexistence of a basic fibroblast growth factor;
(ii) an activity of suppressing hyper degradation of cartilage matrix.

26. A therapeutic agent for a disease caused by suppression of growth of articular chondrocytes, which contains a protein defined in the following (a) or (b) as an active ingredient:
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

27. The therapeutic agent for a disease caused by suppression of growth of articular chondrocytes according to claim 25 or 26, which further contains a basic fibroblast growth factor.

28. A therapeutic agent for a disease caused by suppression of growth of articular chondrocytes, which contains DNA coding for a protein defined in the following (a) or (b):
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

29. The therapeutic agent for a disease caused by suppression of growth of articular chondrocytes according to claim 28, wherein the DNA is DNA defined in the following (c) or (d):
(c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
(d) DNA which is hybridizable with a nucleotide sequence comprising the sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
(i) an activity of growing articular chondrocytes by itself or under coexistence of a basic fibroblast growth factor;
(ii) an activity of suppressing hyper degradation of cartilage matrix.

30. The therapeutic agent for a disease caused by suppression of growth of articular chondrocytes according to claim 28 or 29, which is a drug for gene therapy containing a vector that contains the DNA mentioned in claim 28 or 29 and can be expressed in an animal.

31. A therapeutic agent for a disease caused by hyper degradation of cartilage matrix, which contains a chondromodulin-I protein having activities of the following (i) and (ii) as an active ingredient:
(i) an activity of growing articular chondrocytes by itself or under coexistence of a basic fibroblast growth factor;
(ii) an activity of suppressing hyper degradation of cartilage matrix.

32. A therapeutic agent for a disease caused by hyper degradation of cartilage matrix, which contains a protein defined in the following (a) or (b) as an active ingredient:
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

33. The therapeutic agent for a disease caused by hyper degradation of cartilage matrix according to claim 31 or 32, which further contains a basic fibroblast growth factor.

34. A therapeutic agent for a disease caused by hyper degradation of cartilage matrix, which contains DNA coding for a protein defined in the following (a) or (b):
(a) a protein which has the amino acid sequence of SEQ ID NO: 2, 4 or 6;
(b) a protein which has an amino acid sequence of SEQ ID NO: 2, 4 or 6 including deletion, substitution, insertion or addition of one or several amino acids, and has activities of the following (i) and (ii):
(i) an activity of growing articular chondrocytes by itself or under coexistence of a basic fibroblast growth factor;
(ii) an activity of suppressing hyper degradation of cartilage matrix.

35. The therapeutic agent for a disease caused by hyper degradation of cartilage matrix according to claim 34, wherein the DNA is DNA defined in the following (c) or (d) :
(c) DNA which contains a nucleotide sequence comprising the nucleotide sequence of the nucleotide numbers 2 to 1003 of SEQ ID NO: 1, the nucleotide numbers 2 to 1003 of SEQ ID NO: 3 or the nucleotide numbers 2 to 889 of SEQ ID NO: 5;
(d) DNA which is hybridizable with a nucleotide sequence comprising the sequence of the nucleotide numbers 2 to 1003 of SEQ ID NO.: 1, the nucleotide numbers 2 to 1003 of SEQ ID NO: 3 or the nucleotide numbers 2 to 889 of SEQ ID NO: 5 or a probe that can be prepared from any of these nucleotide sequences under a stringent condition, and codes for a protein having activities of the following (i) and (ii):
(i) an activity of growing articular chondrocytes by itself or under coexistence of a basic fibroblast growth factor;
(ii) an activity of suppressing hyper degradation of cartilage matrix.

36. The therapeutic agent for a disease caused by hyper degradation of cartilage matrix according to claim 34 or 35, which is a drug for gene therapy containing a vector that contains the DNA mentioned in claim 34 or 35 and can be expressed in an animal.
